(19) Europäisches Patentamt
European Patent Office
Office européen des brevets

(11) EP 4 364 731 B1

(12) EUROPEAN PATENT SPECIFICATION

(45) Date of publication and mention of the grant of the patent:
**23.07.2025 Bulletin 2025/30**

(21) Application number: **22383057.1**

(22) Date of filing: **03.11.2022**

(51) International Patent Classification (IPC):
***A61K 31/047*** *(2006.01)* ***A61K 31/728*** *(2006.01)*
***A61P 11/02*** *(2006.01)* ***A61K 9/00*** *(2006.01)*
***A61K 47/26*** *(2006.01)*

(52) Cooperative Patent Classification (CPC):
(C-Sets available)
**A61K 31/728; A61K 9/0043; A61K 31/047; A61K 47/26; A61P 1/00; A61P 11/02;** A61K 9/0078
(Cont.)

# (54) COMPOSITION COMPRISING HYALURONIC ACID AND XYLITOL FOR USE IN THE TREATMENT OF DISRUPTED NASAL MUCOSA AND NASAL EPITHELIAL BARRIER

ZUSAMMENSETZUNG MIT HYALURONSÄURE UND XYLITOL ZUR VERWENDUNG BEI DER BEHANDLUNG VON GESTÖRTER NASENSCHLEIMHAUT UND NASENEPITHELBARRIERE

COMPOSITION AVEC DE L'ACIDE HYALURONIQUE ET DU XYLITOL DESTINÉE À ÊTRE UTILISÉE DANS LE TRAITEMENT DE LA MUQUEUSE NASALE ET DE LA BARRIÈRE ÉPITHÉLIALE NASALE ALTÉRÉE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**08.05.2024 Bulletin 2024/19**

(73) Proprietor: **Laboratorios Cinfa, S.A.**
**31699 Olloki (ES)**

(72) Inventors:
- **MEZQUITA REGUEIRO, Susana**
  **31620 GORRAIZ (ES)**
- **BANDRÉS ARDANAZ, Gabriela**
  **31620 GORRAIZ (ES)**
- **GOÑI DE CERIO, Felipe**
  **48170 ZAMUDIO (ES)**

(74) Representative: **ZBM Patents - Zea, Barlocci & Markvardsen**
**Rambla de Catalunya, 123**
**08008 Barcelona (ES)**

(56) References cited:
**CN-A- 112 773 764** **CN-A- 114 404 466**
**CN-B- 103 735 581** **ES-A1- 2 663 574**

- **MANUELE CASALE ET AL: "Hyaluronic Acid: Perspectives in Upper Aero-Digestive Tract. A Systematic Review", PLOS ONE, vol. 10, no. 6, 29 June 2015 (2015-06-29), pages e0130637, XP055305540, DOI: 10.1371/journal.pone.0130637**
- **CHEN JIANNENG ET AL: "Influence of Hyaluronan Nasal Dressing on Clinical Outcome after Endoscopic Sinus Surgery: A Systematic Review and Meta-Analysis", AMERICAN JOURNAL OF RHINOLOGY & ALLERGY, vol. 31, no. 4, 1 July 2017 (2017-07-01), US, pages 256 - 259, XP093035478, ISSN: 1945-8924, Retrieved from the Internet <URL:http://journals.sagepub.com/doi/full-xml/10.2500/ajra.2017.31.4438> DOI: 10.2500/ajra.2017.31.4438**
- **KIM DO HYUN ET AL: "Effect of Postoperative Xylitol Nasal Irrigation on Patients with Sinonasal Diseases", OTOLARYNGOLOGY AND HEAD AND NECK SURGERY., vol. 160, no. 3, 2 October 2018 (2018-10-02), US, pages 550 - 555, XP093035953, ISSN: 0194-5998, Retrieved from the Internet <URL:https://onlinelibrary.wiley.com/doi/full-xml/10.1177/0194599818802815> DOI: 10.1177/0194599818802815**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SILVA CAROLINE FELIZ FONSECA SEPEDA DA ET AL: "Symptom assessment after nasal irrigation with xylitol in the postoperative period of endonasal endoscopic surgery", BRAZILIAN JOURNAL OF OTORHINOLARYNGOLOGY, vol. 88, no. 2, 1 March 2022 (2022-03-01), pages 243 - 250, XP093035538, ISSN: 1808-8694, DOI: 10.1016/j.bjorl.2020.05.023**
- **KURTARAN HANIFI ET AL: "The effect of different nasal irrigation solutions following septoplasty and concha radiofrequency: a prospective randomized study", BRAZILIAN JOURNAL OF OTORHINOLARYNGOLOGY, vol. 84, no. 2, 22 February 2017 (2017-02-22), pages 185 - 190, XP093035952, ISSN: 1808-8694, DOI: 10.1016/j.bjorl.2017.01.005**


(52) Cooperative Patent Classification (CPC): (Cont.)

C-Sets
**A61K 31/047, A61K 2300/00;**
**A61K 31/728, A61K 2300/00**

## Description

### Technical Field

The present invention relates to the field of sanitary products, more particularly it relates to a composition for use in an integral treatment of disrupted nasal mucosa and epithelial barrier, especially caused by an injury (including surgery), by an infectious process, or by toxic agents, micro-organisms, and/or allergens, where the integrity of the nasal mucosa and epithelial barrier is disrupted partial or totally.

### Background Art

Nasal obstruction is usually the result of anatomical defects (e.g. septal deviation, enlarged nasal turbinates, presence of nasal polyps) and/or environmental factors and is often a symptom of many common nasal diseases such as sinusitis.

Environmental factors such as e.g. allergens, irritants, air-pollutants, virus, bacteria or fungus may stimulate mucous epithelium inducing local inflammation of sinusal mucosa. The resulting inflammation causes approximation of mucosa surfaces, induces ostial obstruction and accumulation of sinus secretion. This favors bacteria proliferation within the paranasal sinuses, and the reduction of the cilia responsible for movement of secretion outside the paranasal sinuses, which creates additional inflammation. When the mucosa and cilia are damaged, the inflammation often becomes chronic.

Sinusitis, also referred to as rhinosinusitis, is defined as the symptomatic inflammation of paranasal sinuses and nasal cavity mucosa. It may evolve into Chronic Rhinosinusitis (CRS) or Recurrent acute rhinosinusitis (RARS). CRS is extremely common and has an estimated prevalence in Europe and in the USA of 10.9% and 11.9%, respectively. On the other hand, RARS occurs when the patient presents four or more episodes of acute rhinosinusitis per year, each lasting at least ten days. Both CRS and RARS patients may also present nasal polyposis.

Nasal polyps are benign (noncancerous) growths of the nasal lining tissues, or nasal mucosa which result from chronic inflammation and are associated with asthma, recurring infection, allergies, drug sensitivity or certain immune disorders. Polyps causes frequent epithelial damage and thickened basal membrane. It may provoke chronic congestion.

Endoscopic sinus surgery (ESS) has been the procedure of choice in treating CRS and nasal polyps, especially in those patients having nasal affections which do not respond to medical treatment. ESS is a nasal intervention which is used to restore sinus ventilation and normal function that allows the treatment of a large number of nasal pathologies.

Although ESS is a minimally invasive surgical technique which requires short surgery times and is associated with fewer surgical complications, it inevitably involves mucosal injury, which may lead, if not properly healed, to severe complications, in particular the formation of nasal crusts in the short-term that may generate an abnormal hyperplasia in the long term, which may require re-intervention

On the other hand, the respiratory epithelium is frequently injured by inhaled toxic agents, micro-organisms, or after physical injury (including a nasal fracture, medicinal treatment or surgery), leading to the alteration of the epithelium barrier integrity. Whatever the source of injury, the response of the respiratory epithelium to an acute injury can be characterized by a succession of cellular events varying from the loss of surface epithelial impermeability to the desquamation of cells from the epithelium. Epithelial damage repair after that is a complex and dynamic process. Although little is known about the physiological basis of the nasal epithelium wound healing, it has been described in *in vivo* models of nasal injury in rabbits and rats that wound healing encompasses the following different phases: hemostasis, inflammation, proliferation, and tissue remodeling.

One of the symptoms of inflammation that is usually observed after surgery is nasal oedema or swelling, which occurs when too much fluid is trapped in the nasal mucosa. Another inflammation symptom is nasal discharge.

Furthermore, it is well known that for an appropriate wound healing and tissue remodeling to take place not only the proliferation of new cells is needed but also a proper migration of the cells to repair the epithelium (re-epithelization) to prevent the wound from becoming chronic. In fact, many *in vivo* models have shown that the first cellular step is the migration of basal cells to cover the area stripped, re-establishment of tight junctions and establishment of a squamous metaplasia, then active proliferation with hyperplasia of the basal and mucous cells followed by progressive differentiation mucous cells into preciliated cells. Further a good re-epithelization is linked with a reduction in the formation of nasal crusts.

Although several treatments have been described for nasal epithelial wound-healing including nasal packings, systemic drug treatments, or nasal sprays, many of these treatments show undesirable side effects, involve patient discomfort. For example, corticosteroids are often used for treating the inflammation after ESS but are known to have an impeding effect on wound healing.

There is still a need to find further therapies for repairing the integrity of the nasal epithelium, especially following an injury or infection, while acting at the same time on associated conditions that seriously affect the quality of life of patients.

## Summary of Invention

The inventors have found that a combination comprising hyaluronic acid, or a salt thereof, and xylitol is particularly useful in the treatment disrupted nasal mucosa and nasal epithelial barrier. In particular, by reducing the formation of nasal crusts in comparison to non-treatment, and more particularly, by reducing nasal oedema and secretion, and increasing cell proliferation and re-epithelization in such a way that the formation of nasal crusts is reduced, in comparison to non-treatment.

Thus, the combination comprising hyaluronic acid, or a salt thereof, and xylitol provides an integral treatment (i.e., a treatment via different routes) for disrupted nasal mucosa and epithelial barrier, in the sense that structural integrity of the mucosa and the epithelial barrier is achieved beyond a restoration of their functionality. Furthermore, the compositions of the invention are easy to use and improve patients' quality of life.

As illustrated in the examples below, the inventors found that a combination comprising hyaluronic acid and xylitol synergistically promoted both cell proliferation and also re-epithelization *in vitro* when compared to the individual components. It is especially remarkable the synergism observed for re-epithelization where the % of re-epithelization for the composition comprising hyaluronic acid and xylitol (41.55%) is 23% higher than the sum of the re-epithelization percentages for hyaluronic acid alone and xylitol alone, being the sum of individual effects 18.6%.

On the other hand, a randomized, placebo-controlled, double-blind clinical trial was conducted assessing the efficacy of a composition comprising sodium hyaluronate and xylitol in patients that have undergone septoplasty with cautery. Turbinate oedema, secretion and nasal crusts were assessed. Oedema and secretion, as determined by using the modified Lund-Kennedy (MLK) scale, statistically significant differences were found between the group treated with the composition of the invention and the placebo group, which was treated with an isotonic solution without sodium hyaluronate and xylitol. Additionally, it was also observed that the number of nasal crusts in treated patients was also reduced, which is direct consequence of the synergistic re-epithelization as observed in the *in vitro* assay. Thus, the group treated with the composition of the invention showed statistically significant reduction of oedema, secretion, and number of nasal crusts versus the placebo group.

As mentioned above, while nasal oedema and discharge are conditions associated with damage to the epithelium and subsequent inflammatory process following an ESS procedure, proliferation and re-epithelization are necessary steps for an appropriate wound healing and avoid post-surgery complications.

Therefore, an aspect of the invention relates to a combination comprising hyaluronic acid, or a pharmaceutically acceptable salt thereof, and xylitol, for use in the treatment of disrupted nasal mucosa and epithelial barrier, wherein the treatment comprises a reduction in the formation of nasal crusts in comparison to non-treatment, more particularly wherein the treatment reduces nasal oedema and secretion, and increases cell proliferation and re-epithelization in such a way that the formation of nasal crusts is reduced, in comparison to non-treatment.

A second aspect of the invention relates to a pharmaceutical composition comprising a therapeutically effective amount of combination comprising hyaluronic acid, or a pharmaceutically acceptable salt thereof, and xylitol, and one or more pharmaceutically acceptable excipients or carriers, for use in the treatment of disrupted nasal mucosa and epithelial barrier, wherein the treatment comprises a reduction in the formation of nasal crusts in comparison to non-treatment, more particularly wherein the treatment reduces nasal oedema and secretion, and increases cell proliferation and re-epithelization in such a way that the formation of nasal crusts is reduced, in comparison to non-treatment.

This aspect may also be formulated as a method of treatment of disrupted nasal mucosa and epithelial barrier, which comprises administering to a subject in need thereof, including a human, a pharmaceutical composition comprising a therapeutically effective amount of hyaluronic acid, or a pharmaceutically acceptable salt thereof and a therapeutically effective amount of xylitol, and one or more pharmaceutically acceptable excipients or carriers, wherein the treatment comprises a reduction in the formation of nasal crusts in comparison to non-treatment, more particularly wherein the treatment reduces nasal oedema and secretion, and increases cell proliferation and re-epithelization in such a way that the formation of nasal crusts is reduced, in comparison to non-treatment.

It also forms part of the invention the use of hyaluronic acid, or a pharmaceutically acceptable salt thereof, and xylitol, for the manufacture of pharmaceutical composition or sanitary product for the treatment of disrupted nasal mucosa and epithelial barrier, wherein the treatment comprises a reduction in the formation of nasal crusts in comparison to non-treatment, more particularly wherein the treatment reduces nasal oedema and secretion, and increases cell proliferation and re-epithelization in such a way that the formation of nasal crusts is reduced, in comparison to non-treatment.

## Brief Description of Drawings

FIG. 1 shows representative images of the scratch after 48 hours of exposure to different concentrations and combinations of Cristalhyal (HA) and Xylitol (X) in HNEpC cell line. The images were taken using the microscope's 4x

objective. The line that marks the limits of the scratch was drawn by ImageJ. Negative control (C-) represents a condition in which no treatment is added, and cells grow in basal medium. Positive control (C+) represents a condition in which cells grow in supplemented medium. Concentration values are expressed as % weight/volume.

FIG. 2 shows representative images of the scratch after 72 hours of exposure to different concentrations and combinations of Cristalhyal (HA) and Xylitol (X) in HNEpC cell line. The images were taken using the microscope's 4x objective. The line that marks the limits of the scratch was drawn by ImageJ. Negative control (C-) represents a condition in which no treatment is added, and cells grow in basal medium. Positive control (C+) represents a condition in which cells grow in supplemented medium. Concentration values are expressed as % weight/volume.

**Detailed description of the invention**

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present invention, any ranges given include both the lower and the upper endpoints of the range.

The term "about" or "around" as used herein refers to a range of values ± 10% of a specified value. For example, the expression "about 10" or "around 10" includes ± 10% of 10, i.e. from 9 to 11.

As mentioned above the present invention relates to a combination comprising hyaluronic acid, or a salt thereof, and xylitol, or alternatively, a pharmaceutical composition comprising a therapeutically effective amount of this combination, and one or more pharmaceutically acceptable excipients or carriers, for use in the treatment of disrupted nasal mucosa and epithelial barrier, in particular by reducing the formation of nasal crusts in comparison to non-treatment, more particularly by reducing nasal oedema and secretion, and increasing cell proliferation and re-epithelization in such a way that the formation of nasal crusts is reduced, in comparison to non-treatment.

For the purposes of the invention, the term "disrupted nasal mucosa and epithelial barrier" refers to a nasal mucosa and epithelial barrier which has suffered from a disruption, i.e., an impairment in the structural (e.g., cell organization) integrity of the mucosa/epithelium that deviates from the normal mucosa/epithelium. The term "normal mucosa/epithelium" refers to a healthy mucosa/epithelium that does not show any detectable indication structural or functional disruption.

The nasal mucosa is a physical barrier to foreign materials and a conditioner for inhaled air that lines the nasal cavity and paranasal sinuses. The nasal epithelium lies on the basement membrane, situated on the lamina propria. The pseudostratified columnar epithelium (respiratory epithelium) is composed of four major types of cells: ciliated cells, nonciliated cells, goblet cells, and basal cells, assuring mucus production and transport, resorption of surface materials, and formation of new epithelial cells.

The expression "in comparison to non-treatment" as used herein refers to the comparison with the evolution of the disrupted nasal mucosa and epithelial barrier without receiving the treatment based on the combination comprising hyaluronic acid, or a salt thereof, and xylitol.

Unlike a treatment for restoring the physiological conditions of the nasal mucosa, the treatment of disrupted nasal mucosa and epithelial barrier involves restoring the integrity of the nasal mucosa and epithelium from a structural point of view, in particular by reducing the formation of nasal crusts in comparison to non-treatment. Nasal crusts affect the structural integrity of the nasal epithelium and mucosa rather than their functionality. By contrast, restoring the physiological conditions of the nasal mucosa refers to restoring the conditions that permit the correct biological function of the nasal mucosa and epithelial barrier in living beings, which are, olfactory function, respiratory function, phonatory function and defense function. More specifically, the defense function is divided in four (1) the mucociliary defense that mainly produces secretions, (2) Ciliary defense, that allows a correct airflow and the release of secretion, (3) inmunological defenses, that it is related with the develop of immunological substances and (4) a defense mediated by the epithelial integrity.

The term "nasal oedema" refers to the degree of inflammation in the nasal mucosa and ephtelium in the nose. The oedema generally is the consequence of the reaction in the tissue that follows after a irritation or injure. The term "turbinate oedema" refers to the degree of inflammation in the anatomical area of the turbinates, smalls structures inside the nose.

The term "secretion" refers to the presence of mucus in the nose, there is no difference between the color but the abundance. The secretion is a reaction that usually is joined with oedema and swelling of the nasal mucosa when a irritation or injure affect the area. In these situations, the goblet cells present in the epithelium tend to increase the secretion of mucus in the nose, as this mucus has a defensive and protective action. For the purposes of the invention, the term secretion also includes discharge, which means when this mucus drips through the anterior or posterior nose.

The term "nasal crusts" refers to hard stuck secretions main located in the septum or turbinates.

The term "cell proliferation" as used herein refers to the replication of epithelial cells, in particular, it refers to the process by which a cell grows and divides to produce two daughter cells. Cell proliferation leads to an increase in cell number. Cell proliferation requires both cell growth and cell division to occur at the same time, such that the average size of cells remains constant in the population.

The term "re-epithelization" as used herein refers to the migration of epithelial cells. Re-epithelialization is defined as a process of covering denuded epithelial surface. The cellular and molecular processes involved in initiation, maintenance, and completion of epithelialization are essential for successful wound closure.

The expression "therapeutically effective amount" as used herein, refers to the amount of hyaluronic acid, or a pharmaceutically acceptable salt thereof or the amount of xylitol which, when administered to a patient, is sufficient to treat, ameliorate or reduce the disruption in the nasal mucosa and epithelial barrier. The specific dose of the compounds to obtain a therapeutic benefit may vary depending on the particular circumstances of the individual patient including, among others, the type of patient, and the nature and stage of the disease or condition.

Xylitol (CAS Registry Number: 87-99-0) is a naturally occurring 5-carbon sugar alcohol having the formula (I).

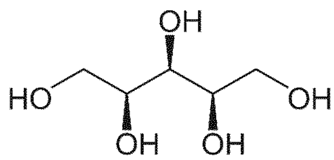

(I)

It is present in small amount in many fruits and vegetables and is produced in the human body during normal metabolism.

Hyaluronic acid (HA, CAS Registry Number: 9004-61-9) is a naturally occurring non sulfated anionic glycosaminoglycan which contains several repeating disaccharide units of formula (II) comprising N-acetyl-D-glucosamine (GlcNac) and D-glucuronic acid (GlcUA).

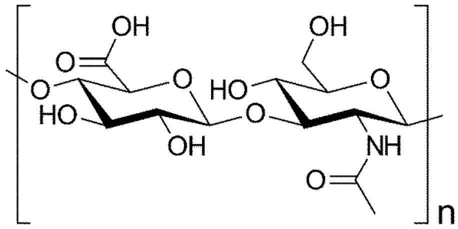

(II)

Hyaluronic acid is widely distributed throughout the body, such as the connective tissue, epithelial tissue, and also neuronal tissues and part of the extracellular matrix.

According to one embodiment, optionally in combination with one or more features of the various embodiments described above or below, the hyaluronic acid of the combination or the composition of the invention is a high molecular weight hyaluronic acid. The term "high molecular weight hyaluronic acid refers to hyaluronic acid having a weight average molecular weight (Mw) equal to or greater than 500kDa, such as from 500 to 2000 kDa.

In one embodiment, optionally in combination with one or more features of the various embodiments described above or below, the hyaluronic acid has a molecular weight from 500 to 2000 kDa, more particularly from 750 to 1800kDa, and even more particularly from 1000 to 1400 kDa.

The molecular weight can be measured by methods well-known in the art such as HPLC in isocratic conditions, using an UV detector.

The hyaluronic acid may be present in the composition of the invention in the form of a pharmaceutically acceptable salt. There is no limitation on the type of hyaluronic acid salt that can be used, as long as the salt is pharmaceutically acceptable when used for therapeutic purposes. The hyaluronic acid and its corresponding salt may differ in some physical properties, but they are equivalent for the purposes of the present invention.

The term "pharmaceutically acceptable salt" refers to a salt appropriate for use in pharmaceutical technology for the preparation of compositions for medical use. In particular, the term "pharmaceutically acceptable salt" encompasses commonly used salts such as, for example, alkali metal salts. The preparation of pharmaceutically acceptable salts of hyaluronic acid can be carried out by methods known in the art. Non-limiting examples of pharmaceutically acceptable

salts of hyaluronic acid suitable for the present invention include inorganic salts such as sodium hyaluronate, magnesium hyaluronate, potassium hyaluronate, zinc hyaluronate and cobalt hyaluronate, as well as organic salts such as tetrabutylammonium hyaluronate.

According to one embodiment, optionally in combination with one or more features of the various embodiments described above or below, the composition of the invention comprises a salt of hyaluronic acid, more particularly sodium hyaluronate (CAS Registry Number: 9067-32-7).

As shown in the examples below, the inventors found that on a human model of nasal epithelium the amount of hyaluronic acid attached to the cellular surface of the nasal epithelium after 120 minutes of its application was higher than in the negative control. Without being bound to theory, it is thought that these results are indicative of the fact that hyaluronic acid *in vivo* will tend to accumulate after its administration on the nasal epithelium for several days of treatment. This means that the composition of the invention will show efficacy in a wide range of concentrations of hyaluronic acid, or a salt thereof, because even the administration of relatively low amounts of it will accumulate and produce in combination with xylitol the advantageous effects described herein.

In one embodiment, optionally in combination with one or more features of the various embodiments described above or below, in the composition of the invention hyaluronic acid, or a salt thereof is present in an amount from 0.01 to 1.5%, more particularly from 0.05 to 0.5%, and even more particularly is about 0.1%, by weight with respect to the total composition weight.

On the other hand, it is expected that xylitol may also be present in the composition of the invention in a wide range of concentrations to obtain the desired effects.

According to one embodiment, optionally in combination with one or more features of the various embodiments described above or below, in the composition of the invention xylitol is present in an amount from 1 to 10%, more particularly from 2 to 8%, and even more particularly is about 5%, by weight with respect to the total composition weight.

In another embodiment, optionally in combination with one or more features of the various embodiments described above or below, in the composition of the invention hyaluronic acid, or a salt thereof is present in an amount from 0.01 to 1.5% by weight, and xylitol is present in an amount from 1 to 10% by weight, wherein the percentages are with respect to the total composition weight. More particularly, hyaluronic acid, or a salt thereof is present in an amount from 0.05 to 0.5% by weight, and xylitol is present in an amount from 2 to 8% by weight, wherein the percentages are with respect to the total composition weight. Even more particularly, hyaluronic acid, or a salt thereof is present in an amount about 0.1% by weight, and xylitol is present in an amount about 5% by weight, wherein the percentages are with respect to the total composition weight.

The pharmaceutical composition of the invention also comprises one or more pharmaceutically acceptable excipients or carriers.

For the purposes of the present invention, the term "pharmaceutically acceptable" excipients or carriers refers to components which are appropriate for use in pharmaceutical technology for the preparation of compositions for medical use. Each component should be "acceptable" in the sense of being compatible with the other ingredients of the composition. When used in contact with the tissue or organ of humans and animals should not have excessive toxicity, irritation, allergic response, immunogenicity, or other problems or complications, commensurate with a reasonable benefit/risk ratio.

The compositions of the invention may be in liquid or gel form. Depending on the desired purpose, the skilled person will know the most appropriate formulation and excipients to be used. Examples of excipients or carriers include, without limitation, solvents, buffers, isotonicity agents, emulsifiers, surfactants, thickeners, buffers, antioxidants, preserving agents, mixtures thereof, and other components known in the state of the art such as e.g. oligoelements. The compositions of the invention are administered nasally.

The term "solvent" refers to a class of liquid organic chemicals of variable lipophilicity and volatility which are used to dissolve, dilute, or disperse materials. Non-limiting examples of solvents include water or seawater.

The term "buffer" as used herein refers to the ability of a system, particularly an aqueous solution, to resist a change of pH on adding acids or bases, or on dilution with a solvent. Non-limiting examples of buffer systems include formate, lactate, benzoate, oxalate, fumarate, acetate, citrate, phosphate, succinate, or tartrate buffers.

The term "isotonicity agent", as used herein refers to an agent that maintains an appropriate osmotic pressure when the composition is applied to the nasal mucosa. Non-limiting examples of isotonicity agent include water-soluble inorganic salts (i.e., mineral salts) such as sodium chloride. Depending on the type and amount of the substances comprised in the formulation, the amount of isotonicity agent included can be adjusted such that the liquid formulation comprising all of the ingredients becomes isotonic or hypertonic. As used herein, the term "isotonic solution" means a solution having substantially the same osmotic pressure as the osmotic pressure of body fluid. Specifically, the term means a solution having an osmotic pressure from 250 to 400 mOsm/L, particularly about 390 mOsm/L. The term "hypertonic solution" means a solution having an osmotic pressure higher than the osmotic pressure of body fluid.

The term "emulsifier" as used herein refers to a material that promotes the formation of intimate mixtures of immiscible liquids by altering interfacial tension.

The term "surfactant" as used herein refers to a material that lowers the surface tension of a liquid and the interfacial tension between two liquids, allowing them to spread more easily. Examples of suitable surface-active agents include, without limitation, nonionic, anionic, cationic, or zwitterionic surfactants.

The term "preservative" as used herein refers to a material that prevents or reduces microbial growth.

The term "thickener" as used herein refers to a material that increases its viscosity without substantially changing its other properties.

According to one embodiment, optionally in combination with one or more features of the various embodiments described above or below, the composition of the invention is a liquid composition, more particularly a solution, and even more particularly an aqueous solution.

In another embodiment, optionally in combination with one or more features of the various embodiments described above or below, the composition of the invention comprises one or more buffers, more particularly a phosphate buffer for example comprising from 0.02 to 1.0% w/v monobasic potassium phosphate and 0.01 to 1.0% w/v dibasic potassium phosphate.

In another embodiment, optionally in combination with one or more features of the various embodiments described above or below, the composition of the invention comprises an isotonicity agent, more particularly the isotonicity agent is sodium chloride. The isotonicity agent may be present in the composition in an amount from 1 to 10%, more particularly from 2 to 5%, by weight with respect to the total composition weight.

In another embodiment, optionally in combination with one or more features of the various embodiments described above or below, the composition of the invention is an isotonic aqueous solution.

In another embodiment, optionally in combination with one or more features of the various embodiments described above or below, the composition of the invention is a hypertonic solution.

In another embodiment, optionally in combination with one or more features of the various embodiments described above or below, the composition of the invention is an aqueous solution which comprises the following components:

a) from 0.01 to 1.5% of hyaluronic acid, or a pharmaceutically acceptable salt thereof, more particularly sodium hyaluronate;
b) from 1 to 10% of xylitol; and
c) a buffer, more particularly a phosphate buffer;

wherein the percentages are given by weight with respect to the total composition weight, provided that the sum of the amounts of the components is less than or equal to 100%.

In another embodiment, optionally in combination with one or more features of the various embodiments described above or below, the composition of the invention is an aqueous solution which comprises the following components:

a) from 0.01 to 1.5% of hyaluronic acid, or a pharmaceutically acceptable salt thereof, more particularly sodium hyaluronate;
b) from 1 to 10% of xylitol;
c) a buffer, more particularly a phosphate buffer; and
d) from 1 to 10% of an isotonicity agent, more particularly a sodium chloride;

wherein the percentages are given by weight with respect to the total composition weight, provided that the sum of the amounts of the components is less than or equal to 100%; and wherein more particularly the aqueous solution comprises seawater.

The composition of the invention can be prepared by methods well-known in the art. For example, in a particular embodiment, hyaluronic acid, or a salt thereof, xylitol may be dissolved in water or seawater containing a buffer and optionally an isotonicity agent.

In another embodiment, optionally in combination with one or more features of the various embodiments described above or below, the composition of the invention is a topical nasal composition, i.e. it is topically administered through the nose. For example, the composition of the invention can be administered by a suitable nebulizer or other nasal devices.

In another embodiment, optionally in combination with one or more features of the various embodiments described above or below, the disruption of the nasal mucosa and epithelial barrier is caused by an injury, more particularly wherein the injury is caused by surgery.

For the purposes of the invention, the term "injury" is a broad term and is used in its ordinary meaning to refer, without limitation, to any tissue damage including a wound, trauma or lesion or any tissue degeneration that is usually inflicted on the body by an external force.

According to one embodiment, optionally in combination with one or more features of the various embodiments described above or below, the invention relates to a combination comprising hyaluronic acid, or a pharmaceutically acceptable salt thereof, and xylitol, for use in the treatment of disrupted nasal mucosa and epithelial barrier, wherein the treatment comprises a reduction in the formation of nasal crusts in comparison to non-treatment, more particularly wherein the treatment comprises a reduction in the formation of nasal crusts in comparison to non-treatment; and wherein the disruption of the nasal mucosa and epithelial barrier is caused by surgery.

According to another embodiment, optionally in combination with one or more features of the various embodiments described above or below, the invention relates to a pharmaceutical composition comprising a therapeutically effective amount of a combination comprising hyaluronic acid, or a pharmaceutically acceptable salt thereof, and xylitol, and one or more pharmaceutically acceptable excipients or carriers, for use in the treatment of disrupted nasal mucosa and epithelial barrier, wherein the treatment comprises a reduction in the formation of nasal crusts in comparison to non-treatment, more particularly wherein the treatment comprises a reduction in the formation of nasal crusts in comparison to non-treatment; and wherein the disruption of the nasal mucosa and epithelial barrier is caused by surgery.

In another embodiment, optionally in combination with one or more features of the various embodiments described above or below, the disruption of the nasal mucosa and epithelial barrier is caused by an infection, for example a viral infection caused by influenza A or Human respiratory syncytial virus (hRSV) or a bacterial infection by *S. pneumoniae, M. catharralis, S. aureus y P. aeruginosa.*

In another embodiment, optionally in combination with one or more features of the various embodiments described above or below, the treatment comprises the administration of a therapeutically effective amount of the composition for a period equal or higher than 28 days, more particularly twice a day. If needed, the treatment may be continued for example by administering a therapeutically effective amount of the composition for treatment periods of 15 days during a total treatment period of three months or according to any other directive given by the physician.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

## Examples

### *In vitro* proliferation (MTT) and re-epithelialization (scratch) assays

*In vitro* studies were performed to assess the proliferation and re-epithelialization activity of compositions containing hyaluronic acid and xylitol as individual components and in combination. Three independent trials were carried out with 4 replicates per experimental trial.

To carry out these studies, the following reagents were used: Airway Epithelial Cell Basal Medium (PROMOCELL; C-21260), Airway Epithelial Cell Growth Medium Supplement Pack (PROMOCELL; C-39160), Fetal Bovine Serum (FBS) (GIBCO; 10270-106), Penicillin/streptomycin (P/S) (GIBCO; 15140-122), Trypsin-EDTA 10X (SIGMA; T4174), Trypsin Inhibitor, soybean (ROCHE; 10109886001), Albumin, from bovine serum (BSA) (SIGMA; A4503), PBS 10X (ROCHE; 11666789001), Hanks' Balanced Salts (SIGMA; H1387), Trypan Blue solution (GIBCO; 15250-061), 3-(4,5-Dimethyl-2-thiazolyl)-2 ,5-diphenyl-2H-tetrazolium bromide (MTT, CAS No 298-93-1, SIGMA; M2128), Dimethyl sulfoxide (DMSO, PANREAC ITW REAGENTS; 161954.1612), Culture-Insert 2 Well 24 (IBIDI; 80241).

Tested compositions

Solutions in basal culture medium of hyaluronic acid (HA, White powder,CAS number 9067-32-7, MW: 1000-1400 kDa, Givaudan France SAS xylitol (Xyl,. White crystalline powder, CAS number 87-99-0 Roquette) or combinations of hyaluronic acid and xylitol at different concentrations were tested.

Regarding hyaluronic acid, insolubility at concentrations above 0.5% w/v prevented the testing of higher concentrations. As for xylitol, a high *in vitro* cytotoxicity at concentrations above 0.5% w/v prevented the testing of higher concentrations.

Experimental system and culture conditions

Human Nasal Epithelial Cells (HNEpC) from a primary cell line isolated from normal human nasal mucosa were purchased form PromoCell (ref: C-12620). The cell line was maintained in supplemented culture medium according to the supplier guidelines and was routinely grown as a monolayer in tissue culture grade flasks. When cells exceeded 70%

confluence (but less than 90%), they were sub-cultured on new culture flasks. All the cell cultures were maintained in standard cell culture conditions (37 °C, 5% $CO_2$ and 95% humidity).

Proliferation assay

MTT, a yellow tetrazole, is reduced to purple formazan by cell metabolic activity. This reduction is proportional to the amount of living cells in the well and, therefore, to cell proliferation over time. Cells cultured in 96-well plates were treated with the test compositions for 24 hours (37 °C, 5% $CO_2$). After 24 hours of incubation, cells were washed with PBS and stained with MTT solution and incubated at 37 °C for 2 hours. At the end of this incubation period, the staining medium was removed and 100 μL of DMSO/well were added to solubilize the colored precipitate. The absorbance was read at 540 nm in the spectrophotometer plate reader. The cell proliferation percentage was calculated relative to the negative control, a condition in which cells were not treated with any product. Cells with treatments and negative control were cultured with basal medium. As positive control, cells were cultured in supplemented medium. The % of cell proliferation was calculated by the following formula:

$$\% \text{ proliferation} = AbT/AbC \times 100$$

AbT = absorbance at 540 nm after 24 hours of treatment
AbC = absorbance at 540 nm of the negative control (24 hours of cells growing in basal medium)

The results are represented in the following table:

Table 1

| Compound concentration (%w/v) | Proliferation | | Number of replicates |
|---|---|---|---|
| | Mean (%) | Standard deviation | |
| Control (negative) | 100.00 | 2.00 | 12 |
| HA 0.5 | 124.81 | 18.50 | 12 |
| Xyl 0.5 | 98.16 | 5.74 | 12 |
| Xyl 0.125 | 99.91 | 5.17 | 12 |
| HA 0.5 + Xyl 0.5 | 157.87 | 16.93 | 12 |
| HA 0.5 + Xyl 0.125 | 135.41 | 20.04 | 12 |
| Control (positive) | 178.40 | 14.92 | 12 |

As can be seen, a synergistic effect was observed on cell proliferation when combining 0.5% HA and 0.5% Xyl since this combination lead to an increase of 57.87% of cell proliferation in relation to negative control, while the addition of their effects separately (24.81 % and 0% respectively) did not reach the combined value. In the same way, the combination of 0.5% HA and 0.125% Xyl also showed a synergistic effect since it gave rise to a greater increase (35.41 %) than the compounds separately (24.81 % and 0% respectively).

Re-epithelialization assay

This assay is based on the premise that cells with higher re-epithelialization rates are able to faster close a wound/scratch generated in a confluent well. Two independent trials were carried out with 3 replicates per experimental trial.

Cells were seeded in both sides of inserts attached on culture wells and were incubated in supplemented medium at 37 °C and 5% $CO_2$. When cells achieved confluence (aprox. 24 hours later), the inserts were removed generating an identical scratch in the cell monolayer of all wells. Then, the wells were washed with 1 mL of PBS and 1 mL of each treatment was added. After 48 hours of incubation, the scratch was photographed. The area of the scratch was calculated using ImageJ software. The percentage of scratch closure (re-epithelialization percentage) was calculated relative to the negative control, a condition in which cells were not treated with any product. Cells with treatments and negative control were cultured with basal medium. As positive control, cells were cultured in supplemented medium. The % of re-epithelialization was calculated by the following formula:

$$\% \text{ re-epithelialization} = 100 - (AT/AC \times 100)$$

AT = area of the scratch after 48 hours of treatment
AC = area of the scratch of the negative control (48 hours of cells growing in basal medium)

The scratch was photographed after 48 hours of exposure to the test compositions. The results are represented in the following table:

Table 2

| Compound concentration (%w/v) | Re-epithelialization | | Number of replicates |
|---|---|---|---|
| | Mean (%) | Standard deviation | |
| Control (negative) | 0.00 | 3.53 | 6 |
| HA 0.5 | 11.85 | 4.27 | 6 |
| Xyl 0.5 | 6.75 | 7.27 | 6 |
| HA 0.5 + Xyl 0.5 | 41.55 | 22.40 | 6 |
| Control (positive) | 100 | 0.00 | 6 |

As can be seen, a synergistic effect was observed on re-epithelialization when combining 0.5% HA and 0.5% Xyl. This combination (0.5 HA + 0.5 Xyl) lead to an increase of the 41.55% of cell re-epithelialization in relation to negative control, while the addition of their effects separately (11.85% and 6.75% respectively) did not reach the combined value. In addition, the scratch was photographed after 72 hours of exposure to the tested compositions. It was observed that cells treated with the combination of HA 0.5% and Xyl 0.5% closed the scratch completely, while HA 0.5% and Xyl 0.5% separately did not.

The evolution of the scratch was observed after 48 hours of exposure to different concentrations and combinations of Cristalhyal (HA) and Xylitol (X) in HNEpC cell line using a microscope's 4x objective. Representative images are shown in FIG. 1. The line that marks the limits of the scratch was drawn by ImageJ. Negative control (C-) represents a condition in which no treatment is added, and cells grow in basal medium. Positive control (C+) represents a condition in which cells grow in supplemented medium. As can be seen the combination of hyaluronic acid and xylitol almost completely closed the scratch after 48h.

In addition, the synergistic conditions were followed for another 24 hours, and the scratch was photographed after 72 hours of exposure to the compounds. Images are shown in FIG. 2. Cells treated with the combination of HA 0.5% and Xyl 0.5% closed the scratch completely, while HA 0.5% and Xyl 0.5% separately did not.

As can be seen in the cell re-epithelialization and proliferation assays, a clear synergy between xylitol and hyaluronic acid was observed. The hypothesis of this synergistic effect would be understood as a physical effect where the combination of the two compounds under study, xylitol and hyaluronic acid, would produce a cell support or scaffolding effect that would facilitate greater cell proliferation and better tissue re-epithelialization by the cells of the nasal epithelium.

**Study of the interaction of hyaluronic acid (HA) on a human model of nasal epithelium**

The aim of this in vitro study was to test how hyaluronic acid (HA) behaved and interacted in a human model of nasal epithelium.

Experimental system

The experimental system used in this study was a 3D model of fully differentiated human nasal epithelium provided by Epithelix Sas: MucilAir™ Nasal Cat No.: EP01MD.

It is a human epithelium reconstituted *in vitro* with cells isolated from the nasal cavity. It was received as a fully differentiated epithelia in transwells of 6.5 mm of diameter, with a pore size of 0.4 μm. Transwells received at Gaiker were placed, according to the provider's instructions, in a standard tissue culture incubator (a 37°C and 5% CO2) for 7 days, with 0.7 ml of MucilAir™ medium basolateral replacement every 2-3 days.

Previously, before carrying out the study in human nasal epithelium, cell viability determination via MTT assay was carried out to properly adjust the concentrations of the test elements to the in vitro nasal uptake assay, a viability assay was performed in A549 cells (human alveolar basal epithelial cells). Data not shown.

During the assay, transepithelial/transendothelial electrical resistance (TEER) measurements were performed

to test the integrity of the epithelial barrier. As well as the determination of TEER. A Lucifer yellow assay was also performed to check the integrity of the epithelial barrier. (Data not shown. TEER and lucifer yellow values were adequate, no alteration in barrier integrity was observed).

Once incubation with the test elements was completed, the epithelia of the inserts were incubated with Lucifer Yellow to check the permeability of Lucifer Yellow in the epithelium as a means of barrier integrity. (Data not shown, all results were adequate, no permeability disruption was observed).

Hyaluronic acid determination

Hyaluronic acid in the samples taken was determined by a quantitative sandwich enzyme immunoassay (ELISA) technique: Hyaluronan Quantikine ELISA Kit, R&D Systems DHYAL0, following the manufacturer's instructions.

Nasal epithelia transwells were incubated with HA at 25% for up to 120 minutes in order to measure the transportation of hyaluronic acid contained through the epithelium following its application. In the same way, as the epithelium already contains hyaluronic acid, the transport of naturally occurring hyaluronic acid, was analyzed by using a negative control transwells group, which did not receive any treatment (they were incubated only with the transport media for 120 minutes). Once the hyaluronic acid interaction assay was finished the nasal epithelia surfaces were thoroughly washed with phosphate-buffered saline (PBS). The hyaluronic acid content in the washing buffer was then determined. Results of the amount of hyaluronic acid attached to the epithelia after application of HA is shown below. As indicated above, the negative control (nasal epithelia where HA was not added) was also included in the study due to the intrinsic presence of hyaluronic acid in the cells of nasal epithelium.

| Hyaluronic acid in Nasal Epithelia Surface | |
|---|---|
| HA | 333.29 |
| Neg. Control | 16.61 |

As can be seen the amount of hyaluronic acid attached to the cellular surface is higher than in the negative control.

The results indicated that hyaluronic acid has a high adherence to the nasal epithelium, so it is believed that in a multidose study o in a clinical trial hyaluronic acid will accumulate in the nasal epithelium. This will facilitate the combination of xylitol and hyaluronic acid to produce the synergy previously observed.

**Clinical trial**

A randomized, placebo-controlled, double-blind, parallel-group clinical trial was performed to evaluate the clinical efficacy and the quality of life of patients requiring septoplasty with cautery, with or without submucosal resection of the inferior turbinates, using a composition comprising sodium hyaluronate and xylitol. The tolerability of the product and the clinical evolution post-surgery were also evaluated as well as the patients' satisfaction.

The clinical study had an active duration per patient of 35 days: 1 screening visit, 4 treatment weeks after surgery and 1 follow-up week. 58 subjects were recruited in the study, 55 were enrolled (compliance with the eligibility criteria) and 47 completed the study. The evaluation visits were 5: visit 1: pre-operation day, visit 2: day 0 post surgery (D0, start treatment on the same day), visit 3: day 7 post surgery (D7), visit 4: day 14 post surgery (D14), and visit 5: day 28 post surgery.

During the treatment time, the participants carried out the treatment as follows: each patient used either the product under study or the placebo product in a blinded manner for both the patient and the investigator for 28 days for twice a day (morning and evening), one vial per nostril at each administration, both products being in single-dose vial format. One group of patients received the product under study and the other a placebo.

The product (isotonic solution containing sodium hyaluronate: 0.1% w/v, xylitol: 5% w/v, potassium phosphate monobasic: 0.02 to 1.0% w/v, potassium phosphate dibasic: 0.01 to 1.0% w/v, and water) or the placebo (isotonic solution containing sodium chloride: 0.9% w/v, potassium phosphate monobasic: 0.02 to 1.0% w/v, potassium phosphate dibasic: 0.01 to 1.0% w/v, and water) were administered by means of the MAD Nasal™ medical device (nebulizer).

The following tests were performed:

- Modified Lund-Kennedy endoscopic scale (MLK)

  Oedema: 0 = absent, 1 = mild, 2 = severe or beyond middle meatus
  Secretion: 0 = no secretion, 1 = clear, thin secretion, 2 = thick, purulent secretion

No adverse effects or serious product deficiencies were recorded throughout the study.

For both placebo and product groups, the differences at each experimental time with respect to baseline were calculated: D7-D0, D14-D0 and D28-D0. A descriptive statistical analysis of the quantitative biometric variables (MLK secretion Scale and MLK oedema Scale) was performed, including basic descriptive parameters (central tendency and variation) and absolute variation with respect to placebo.

Linear mixed-effects models (LMM) were fitted. If the data followed a normal distribution and could not be fitted by linear mixed-effects models, an unpaired samples Student's t-test was used. On the other hand, if the data did not follow a normal distribution, the unpaired samples Wilcoxon signed-rank test was used.

Significance level is established in $p<0.05$ for each statistical test performed in this study.

MLK oedema scale

The following table shows the descriptive statistics of the differences Di-D0 in the MLK oedema scale for the comparison of product with placebo, including the average, the standard deviation and the percentage of absolute variation with respect to placebo.

Table 3

| MLK oedema scale | | | | | | |
|---|---|---|---|---|---|---|
| | **Placebo group** | | | **Product group** | | |
| | **D7- D0** | **D14 - D0** | **D28 - D0** | **D7- D0** | **D14 - D0** | **D28 - D0** |
| Average | 0.45 | -0.19 | -0.32 | -0.15 | -0.63 | -0.92 |
| Standard deviation | 0.67 | 0.75 | 0.57 | 0.77 | 0.74 | 0.64 |
| % of absolute variation respect to placebo | - | - | - | -133% | -231% | -189% |

The difference D7-D0 in the MLK oedema scale in the product group was an average of 133% lower in relation to placebo. The difference D14-D0 in the MLK oedema scale in the product group was an average of 231% lower in relation to placebo. The difference D28-D0 in the MLK oedema scale in the product group was an average of 189% lower in relation to placebo. The differences D7-D0 and D28-D0 were statistically significant with a p value lower than 0.05 (Wilcoxon Signed-Rank Test).

MLK secretion scale

The following table shows the descriptive statistics of the differences Di-D0 in the MLK secretion scale for the comparison of product with placebo, including the average, the standard deviation and the percentage of absolute variation with respect to placebo.

Table 5

| MLK secretion scale | | | | | | |
|---|---|---|---|---|---|---|
| | **Placebo group** | | | **Product group** | | |
| | **D7- D0** | **D14 - D0** | **D28 - D0** | **D7- D0** | **D14 - D0** | **D28 - D0** |
| Average | 0.64 | 0.24 | 0.05 | 0.00 | -0.04 | -0.44 |
| Standard deviation | 0.66 | 0.62 | 0.65 | 0.88 | 0.81 | 0.87 |
| % of absolute variation respect to placebo | - | - | - | -100% | -116% | -1068% |

The difference D7-D0 in the MLK secretion scale in the product group was an average of 100% lower in relation to placebo. The difference D14-D0 in the MLK secretion scale in product group was an average of 116% lower in relation to placebo. The difference D28-D0 in the MLK secretion scale in product group was an average of 1068% lower in relation to placebo. The difference D7-D0 was statistically significant with a p value lower than 0.05 (Wilcoxon Signed-Rank Test).

Analysis of the number of crusts

The following tables show the absolute and relative frequency of crusts in the placebo and treatment groups at

times D0, D7, D14, and D28. Additionally, the increased risk ratio with a 95% confidence interval for D7 and D14 is also shown. For the calculation of the relative risk, having zero or one crust was considered as not showing disease, while having two or more crusts was considered as showing disease. For the first experimental time (D0) it was not possible to construct the relative risk interval as in both treatment groups there were no patients with two or more crusts. For experimental time (D28) it was not possible to construct the relative risk interval, as there were no patients treated with product with two or more crusts.

Experimental time D0

| GRADE | PRODUCT GROUP | | | PLACEBO GROUP | | |
|---|---|---|---|---|---|---|
| | Frequencies (%) | | TOTAL | Frequencies (%) | | TOTAL |
| | 0-1 | 2 or + | | 0-1 | 2 or + | |
| n | 52 | 0 | 52 | 42 | 0 | 42 |
| % | 100% | 0% | 100% | 100% | 0% | 100% |

At experimental time D0, 100% of the patients treated with product had 0 or 1 crust (52 out of 52). As for the patients treated with placebo, 100% had 0 or 1 crust (42 out of 42).

Experimental time D7

| GRADE | PRODUCT GROUP | | | PLACEBO GROUP | | |
|---|---|---|---|---|---|---|
| | Frequencies (%) | | TOTAL | Frequencies (%) | | TOTAL |
| | 0-1 | 2 or + | | 0-1 | 2 or + | |
| n | 41 | 9 | 50 | 28 | 14 | 42 |
| % | 82% | 18% | 100% | 67% | 33% | 100% |

At experimental time D7, 82% of the patients treated with product had 0 or 1 crust (41 out of 50), while 18% had 2 or more crusts (9 out of 50). As for the patients treated with placebo, 67% had 0 or 1 crust (28 out of 42), while 33% had 2 or more crusts (14 out of 42).

| TIME D7 | Frequency | | Increased risk ratio |
|---|---|---|---|
| | 2 or + crust | 0-1 crust | |
| Product group | 9 | 41 | 0.54 (0.26, 1.12) |
| Placebo Group | 14 | 28 | |

At experimental time D7, in patients treated with product there is a 46% reduction in the risk of developing 2 or more crusts compared to the placebo group, but there is no scientific evidence that treatment with product is a protective agent against developing 2 or more crusts (RR =0.54; IC 95%: 0.26-1.12).

Experimental time D14

| GRADE | PRODUCT GROUP | | | PLACEBO GROUP | | |
|---|---|---|---|---|---|---|
| | Frequencies (%) | | TOTAL | Frequencies (%) | | TOTAL |
| | 0-1 | 2 or + | | 0-1 | 2 or + | |
| n | 44 | 4 | 48 | 28 | 12 | 40 |
| % | 92% | 8% | 100% | 67% | 33% | 100% |

At experimental time D14, 92% of the patients treated with product had 0 or 1 crust (44 out of 48), while 8% had 2 or more crusts (4 out of 48). As for the patients treated with placebo, 67% had 0 or 1 scab (28 out of 40), while 33% had 2 or more crusts (12 out of 40).

| TIME D14 | Frequency | | Increased risk ratio |
|---|---|---|---|
| | 2 or + crust | 0-1 crust | |
| Product group | 4 | 44 | 0.28 (0.10, 0.79) |
| Placebo Group | 12 | 28 | |

At experimental time D14, in patients treated with product there is a 72% reduction in the risk of developing 2 or more crusts compared to the Placebo group. Treatment with product is a significant protective factor against developing 2 or more crusts at experimental time D14 (RR =0.28; IC 95%: 0.10-0.79).

Experimental time D28

| GRADE | PRODUCT GROUP | | | PLACEBO GROUP | | |
|---|---|---|---|---|---|---|
| | Frequencies (%) | | TOTAL | Frequencies (%) | | TOTAL |
| | 0-1 | 2 or + | | 0-1 | 2 or + | |
| n | 36 | 0 | 36 | 18 | 8 | 26 |
| % | 100% | 0% | 100% | 69% | 31% | 100% |

At experimental time D28, 100% of the patients treated with product had 0 or 1 crust (36 out of 36). As for the patients treated with placebo, 69% had 0 or 1 crust (18 out of 26), while 31% had 2 or more crusts (8 out of 26).

As previously said, oedema and secretions are conditions associated with damage of the mucosa and nasal epithelium. The oedema (inflammation) and secretions (increase of mucous in the evaluated area) are processes that if they continue in time, can cause a chronic impaired nasal mucosa and epithelial barrier, leading a pathological mucosa and nasal epithelia. In addition, the swelling of the nasal structures during the damage and the presence of crusts, can promote the possibility of contact between the opposite surfaces inside the nose or form fibrous tissue that can block the normal airflow through the nose increasing the discomfort of patients.

In this clinical study, the analysis of oedema, secretions and number of crusts showed that in the group treated with the combination of HA+Xylitol versus placebo, there was a reduction on the degree of the oedema or secretion compared to the placebo group. Furthermore, the reduction in the number of crusts was also evident in the treated group with HA+Xylitol and it was shown that there was a significant protective factor against developing crusts.

All these clinical processes are intimately linked to the correct proliferation and re-epithelization process at cellular level. These steps are necessary for an appropriate wound healing and avoid post-surgery complications as a chronic inflammation or the promotion of fibrous tissue. The reduction in the nasal crusts formed is an indirect proof of good re-epithelization and healing of the impaired mucosa and nasal epithelium.

## Claims

1. A combination comprising hyaluronic acid, or a pharmaceutically acceptable salt thereof, and xylitol, for use in the

treatment of disrupted nasal mucosa and nasal epithelial barrier, wherein the treatment comprises a reduction in the formation of nasal crusts in comparison to non-treatment.

**2.** A pharmaceutical composition comprising a therapeutically effective amount of a combination comprising hyaluronic acid, or a pharmaceutically acceptable salt thereof, and xylitol, and one or more pharmaceutically acceptable excipients or carriers, for use in the treatment of disrupted nasal mucosa and nasal epithelial barrier, wherein the treatment comprises a reduction in the formation of nasal crusts in comparison to non-treatment.

**3.** The combination for use according to claim 1 or the pharmaceutical composition for use according to claim 2, wherein the treatment reduces nasal oedema and secretion, and increases cell proliferation and re-epithelization in such a way that the formation of nasal crusts is reduced, in comparison to non-treatment.

**4.** The combination or the pharmaceutical composition for use according to any of claims 1-3, wherein the hyaluronic acid, or a salt thereof is sodium hyaluronate.

**5.** The combination or the pharmaceutical composition for use according to any of claims 1-4, wherein the hyaluronic acid, or a salt thereof has an average molecular weight from 500 to 2000 kDa.

**6.** The pharmaceutical composition for use according to any of claims 2-5, wherein the hyaluronic acid, or a salt thereof is present in an amount from 0.01 to 1.5% by weight with respect to the total composition weight.

**7.** The pharmaceutical composition for use according to any of claims 2-6, wherein xylitol is present in an amount from 1 to 10% by weight, respectively, with respect to the total composition weight.

**8.** The pharmaceutical composition for use according to any of claims 2-7, which is an aqueous solution.

**9.** The pharmaceutical composition for use according to claim 8, wherein the aqueous solution further comprises one or more buffers.

**10.** The pharmaceutical composition for use according to any of claims 8-9, which is an isotonic aqueous solution or a hypertonic solution.

**11.** The pharmaceutical composition for use according to any of claims 2-10 which is a topical nasal composition.

**12.** The pharmaceutical composition for use according to any of claims 2-11, wherein the composition comprises the following components:

a) from 0.01 to 1.5% of hyaluronic acid, or a pharmaceutically acceptable salt thereof;
b) from 1 to 10% of xylitol; and
c) a buffer, more particularly a phosphate buffer;
or alternatively, the composition comprises the following components:

a) from 0.01 to 1.5% of hyaluronic acid, or a pharmaceutically acceptable salt thereof;
b) from 1 to 10% of xylitol;
c) a buffer, more particularly a phosphate buffer; and
d) from 1 to 10% of an isotonicity agent;

wherein the percentages are given by weight with respect to the total composition weight provided that the sum of the amounts of the components is less than or equal to 100%.

**13.** The combination or the pharmaceutical composition for use according to any of claims 1-12, wherein the disruption of the nasal mucosa and epithelial barrier is caused by an injury.

**14.** The combination or the pharmaceutical composition for use according to claim 13, wherein the injury is caused by surgery.

**15.** The combination or the pharmaceutical composition for use according to any of claims 1-14, wherein the disruption of the nasal mucosa and epithelial barrier is caused by an infectious process.

**Patentansprüche**

1. Eine Kombination umfassend Hyaluronsäure oder ein pharmazeutisch akzeptables Salz davon und Xylit, zur Verwendung bei der Behandlung der gestörten Nasenschleimhaut und nasalen Epithelbarriere, wobei die Behandlung eine Verringerung der Bildung von Nasenkrusten im Vergleich zur Nichtbehandlung umfasst.

2. Eine pharmazeutische Zusammensetzung umfassend eine therapeutisch wirksame Menge einer Kombination umfassend Hyaluronsäure oder ein pharmazeutisch akzeptables Salz davon und Xylit und einen oder mehrere pharmazeutisch akzeptable Hilfsstoffe oder Trägersubstanzen zur Verwendung bei der Behandlung der gestörten Nasenschleimhaut und nasalen Epithelbarriere, wobei die Behandlung eine Verringerung der Bildung von Nasenkrusten im Vergleich zur Nichtbehandlung umfasst.

3. Die Kombination zur Verwendung nach Anspruch 1 oder die pharmazeutische Zusammensetzung zur Verwendung nach Anspruch 2, wobei die Behandlung das nasale Ödem und die Sekretionen reduziert und die Zellproliferation und die Reepithelisierung derart erhöht, dass die Bildung von Nasenkrusten im Vergleich zur Nichtbehandlung reduziert wird.

4. Die Kombination oder die pharmazeutische Zusammensetzung zur Verwendung nach einem der Ansprüche 1 bis 3, wobei die Hyaluronsäure oder ein Salz davon Natriumhyaluronat ist.

5. Die Kombination oder die pharmazeutische Zusammensetzung zur Verwendung nach einem der Ansprüche 1 bis 4, wobei die Hyaluronsäure oder ein Salz davon ein durchschnittliches Molekulargewicht von 500 bis 2000 kDa hat.

6. Die pharmazeutische Zusammensetzung zur Verwendung nach einem der Ansprüche 2 bis 5, wobei die Hyaluronsäure oder ein Salz davon in einer Menge von 0,01 bis 1,5 Gew.- %, bezogen auf das gesamte Gewicht der Zusammensetzung, vorhanden ist.

7. Die pharmazeutische Zusammensetzung zur Verwendung nach einem der Ansprüche 2 bis 6, wobei der Xylit jeweils in einer Menge von 1 bis 10 Gew.- %, bezogen auf das gesamte Gewicht der Zusammensetzung, vorhanden ist.

8. Die pharmazeutische Zusammensetzung zur Verwendung nach einem der Ansprüche 2 bis 7, welche eine wässrige Lösung ist.

9. Die pharmazeutische Zusammensetzung zur Verwendung nach Anspruch 8, wobei die wässrige Lösung ferner einen oder mehrere Puffer umfasst.

10. Die pharmazeutische Zusammensetzung zur Verwendung nach einem der Ansprüche 8 bis 9, welche eine isotonische wässrige Lösung oder eine hypertonische Lösung ist.

11. Die pharmazeutische Zusammensetzung zur Verwendung nach einem der Ansprüche 2 bis 10, welche eine topische nasale Zusammensetzung ist.

12. Die pharmazeutische Zusammensetzung zur Verwendung nach einem der Ansprüche 2 bis 11, wobei die Zusammensetzung die folgenden Bestandteile umfasst:

    a) von 0,01 bis 1,5 % Hyaluronsäure oder ein pharmazeutisch akzeptables Salz davon;
    b) von 1 bis 10 % Xylit; und
    c) einen Puffer, insbesondere einen Phosphatpuffer;
    oder, ersatzweise, umfasst die Zusammensetzung die folgenden Bestandteile:

        a) von 0,01 bis 1,5 % Hyaluronsäure oder ein pharmazeutisch akzeptables Salz davon;
        b) von 1 bis 10 % Xylit;
        c) einen Puffer, insbesondere einen Phosphatpuffer; und
        d) von 1 bis 10 % eines Isotonizitätsmittels;

    wobei die Prozentsätze als Gewichtsprozente in Bezug auf das gesamte Gewicht der Zusammensetzung angegeben sind, unter der Voraussetzung, dass die Summe der Mengen der Bestandteile kleiner als oder gleich 100 % ist.

13. Die Kombination oder die pharmazeutische Zusammensetzung zur Verwendung nach einem der Ansprüche 1 bis 12, wobei die Störung der Nasenschleimhaut und der Epithelbarriere durch eine Verletzung verursacht wird.

14. Die Kombination oder die pharmazeutische Zusammensetzung zur Verwendung nach Anspruch 13, wobei die Verletzung durch eine Operation verursacht wird.

15. Die Kombination oder die pharmazeutische Zusammensetzung zur Verwendung nach einem der Ansprüche 1 bis 14, wobei die Störung der Nasenschleimhaut und der Epithelbarriere durch einen infektiösen Prozess verursacht wird.

## Revendications

1. Une combinaison comprenant de l'acide hyaluronique, ou un sel pharmaceutiquement acceptable de celui-ci, et du xylitol, pour son utilisation dans le traitement de la muqueuse nasale et de la barrière épithéliale nasale perturbées, dans laquelle le traitement comprend une réduction de la formation de croûtes nasales par rapport au non-traitement.

2. Une composition pharmaceutique comprenant une quantité thérapeutiquement efficace d'une combinaison comprenant de l'acide hyaluronique, ou un sel pharmaceutiquement acceptable de celui-ci, et du xylitol, et un ou plusieurs excipients ou véhicules pharmaceutiquement acceptables, pour son utilisation dans le traitement de la muqueuse nasale et de la barrière épithéliale nasale perturbées, dans laquelle le traitement comprend une réduction de la formation de croûtes nasales par rapport au non-traitement.

3. La combinaison pour son utilisation selon la revendication 1 ou la composition pharmaceutique pour son utilisation selon la revendication 2, dans laquelle le traitement réduit l'oedème nasal et les sécrétions, et augmente la prolifération cellulaire et la ré-épithélisation de telle sorte que la formation de croûtes nasales est réduite, par rapport au non-traitement.

4. La combinaison ou la composition pharmaceutique pour son utilisation selon l'une quelconque des revendications 1 à 3, dans laquelle l'acide hyaluronique, ou un sel de celui-ci est l'hyaluronate de sodium.

5. La combinaison ou la composition pharmaceutique pour son utilisation selon l'une quelconque des revendications 1 à 4, dans laquelle l'acide hyaluronique, ou un sel de celui-ci a un poids moléculaire moyen de 500 à 2000 kDa.

6. La composition pharmaceutique pour son utilisation selon l'une quelconque des revendications 2 à 5, dans laquelle l'acide hyaluronique, ou un sel de celui-ci, est présent en une quantité de 0,01 à 1,5 % en poids par rapport au poids total de la composition.

7. La composition pharmaceutique pour son utilisation selon l'une quelconque des revendications 2 à 6, dans laquelle le xylitol est présent en une quantité de 1 à 10 % en poids, respectivement, par rapport au poids total de la composition.

8. La composition pharmaceutique pour son utilisation selon l'une quelconque des revendications 2 à 7, qui est une solution aqueuse.

9. La composition pharmaceutique pour son utilisation selon la revendication 8, dans laquelle la solution aqueuse comprend en outre un ou plusieurs tampons.

10. La composition pharmaceutique pour son utilisation selon l'une quelconque des revendications 8 à 9, qui est une solution aqueuse isotonique ou une solution hypertonique.

11. La composition pharmaceutique pour son utilisation selon l'une quelconque des revendications 2 à 10, qui est une composition nasale topique.

12. La composition pharmaceutique pour son utilisation selon l'une quelconque des revendications 2 à 11, dans laquelle la composition comprend les composants suivants :

    a) de 0,01 à 1,5 % d'acide hyaluronique, ou un sel pharmaceutiquement acceptable de celui-ci ;
    b) de 1 à 10 % de xylitol ; et
    c) un tampon, plus particulièrement un tampon phosphate ;

ou, en variante, la composition comprend les composants suivants :

a) de 0,01 à 1,5 % d'acide hyaluronique, ou un sel pharmaceutiquement acceptable de celui-ci ;
b) de 1 à 10 % de xylitol ;
c) un tampon, plus particulièrement un tampon phosphate ; et
d) de 1 à 10% d'un agent d'isotonicité ;

dans laquelle les pourcentages sont donnés en poids par rapport au poids total de la composition à condition que la somme des quantités des composants soit inférieure ou égale à 100 %.

**13.** La combinaison ou la composition pharmaceutique pour son utilisation selon l'une quelconque des revendications 1 à 12, dans laquelle la perturbation de la muqueuse nasale et de la barrière épithéliale est causée par une blessure.

**14.** La combinaison ou la composition pharmaceutique pour son utilisation selon la revendication 13, dans laquelle la blessure est causée par une procédure chirurgicale.

**15.** La combinaison ou la composition pharmaceutique pour son utilisation selon l'une quelconque des revendications 1 à 14, dans laquelle la perturbation de la muqueuse nasale et de la barrière épithéliale est causée par un processus infectieux.

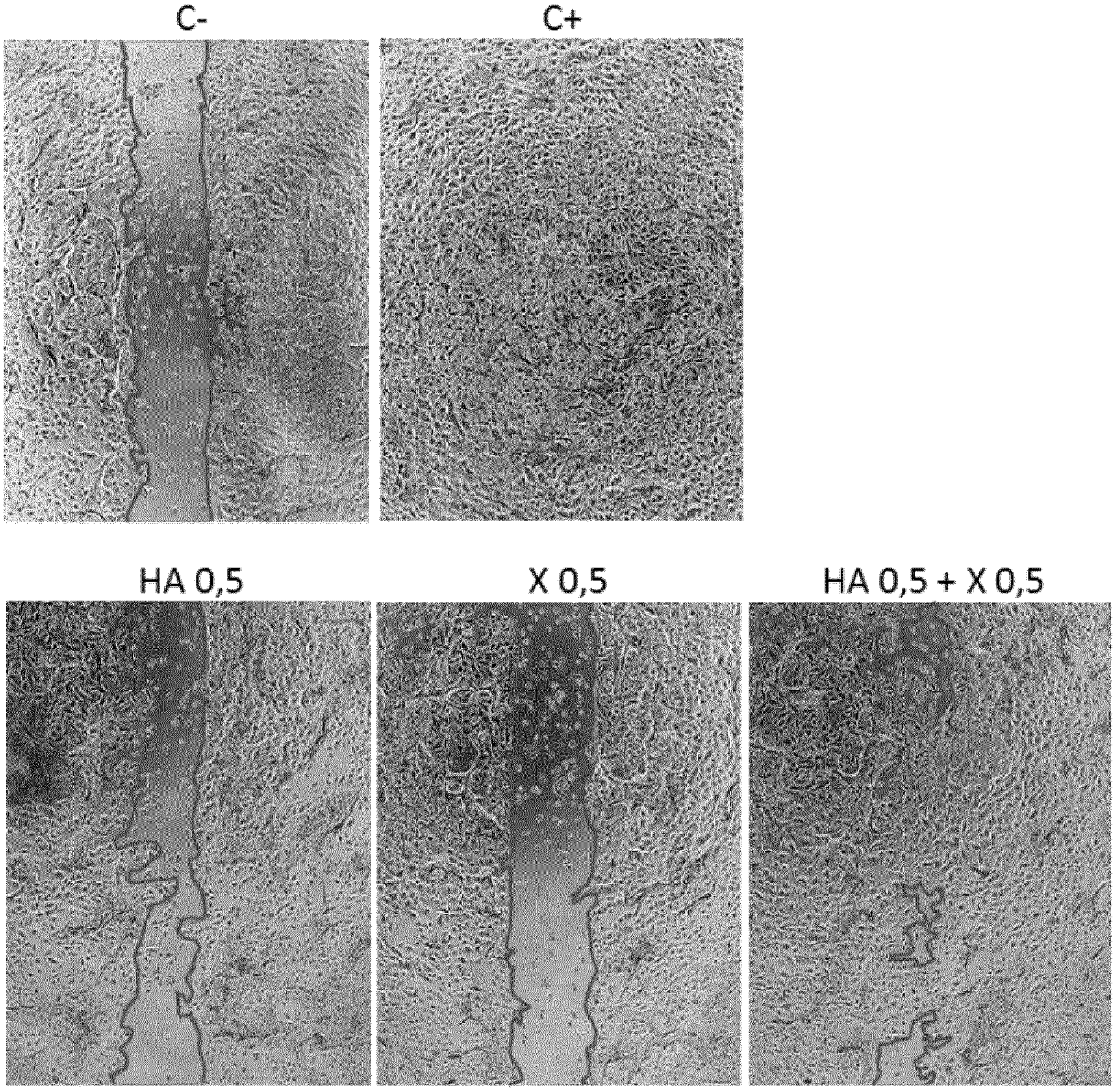


FIG. 1

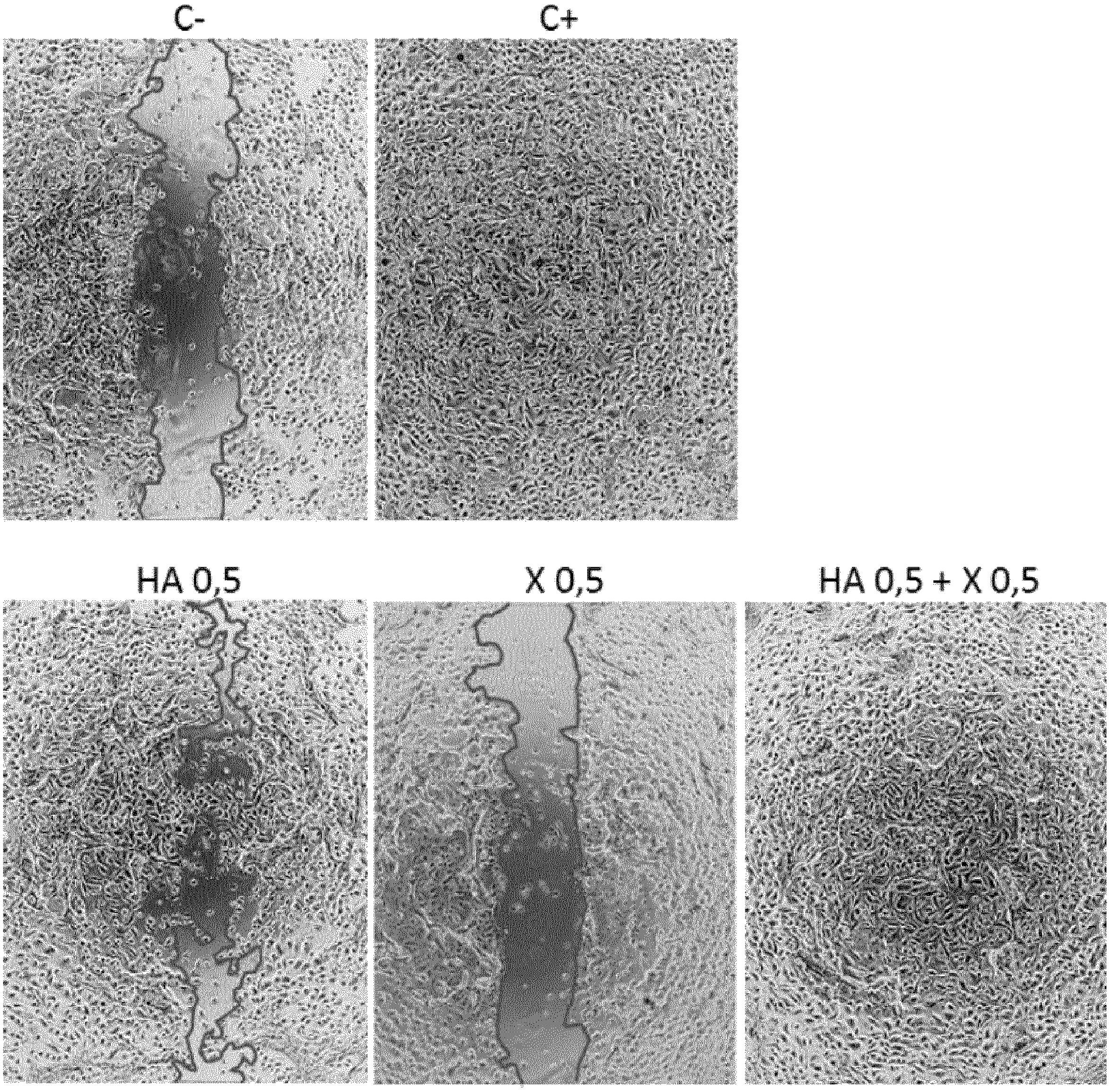


FIG. 2